# EUROPEAN PATENT SPECIFICATION

(11) **EP 3 534 373 B1**
(45) Date of publication and mention of the grant of the patent: **11.03.2026**
(21) Application number: 19178174.9
(22) Date of filing: 04.06.2019
(51) Int. Cl.: G16H 50/30, G16H 20/70, A61B 5/00, A61B 5/08, A61B 5/024, A61B 5/113, A61B 5/16, A61B 5/0205

(54) **SYSTEM FOR MEASURING STRESS LEVEL**
SYSTEM ZUR MESSUNG DES STRESSNIVEAUS
SYSTÈME DE MESURE DU NIVEAU DE STRESS

(43) Date of publication of application: 04.09.2019
(73) Proprietor: Polar Electro Oy, 90440 Kempele (FI)
(72) Inventor: Celka, Patrick, 90440 Kempele (FI); Martinmäki, Kaisu, 90440 Kempele (FI); Tuulari, Esa, 90440 Kempele (FI); Santaniemi, Nuutti, 90440 Kempele (FI); Ahola, Riikka, 90440 Kempele (FI); Korhonen, Topi, 90440 Kempele (FI); Turpeinen, Inka, 90440 Kempele (FI); Rahko, Juho, 90440 Kempele (FI); Rönnberg, Lotta, 90440 Kempele (FI)
(74) Representative: Kolster Oy Ab

(56) References cited:
- WO-A1-2009/104127
- WO-A1-2017/212120
- WO-A1-2019/077304
- JP-A- 2013 128 659
- US-A1- 2007 056 582
- US-A1- 2015 282 723
- PATRICK CELKA ET AL: "Pulse Wave Harmony: Ancient Wisdoms for Modern Age", 15 June 2019 (2019-06-15), XP055610237, Retrieved from the Internet <URL:https://www.researchgate.net/profile/Patrick_Celka/publication/333918089_Pulse_Wave_Harmony_Ancient_Wisdoms_for_Modern_Age/links/5d0cb12a458515c11ceafe52/Pulse-Wave-Harmony-Ancient-Wisdoms-for-Modern-Age.pdf> [retrieved on 20190801], DOI: 10.20944/preprints201906.0204.v1

## Description

### TECHNICAL FIELD

The invention relates to measurement devices and algorithms and, in particular, to a system for measuring a stress level of a human.

### TECHNICAL BACKGROUND

An autonomic nervous system (ANS) of a human controls involuntary functions of a body, including the function of a cardiovascular system. The ANS consists of two branches, a sympathetic nervous system (SNS) and a parasympathetic nervous system (PNS). These two branches generally exert opposing effects on a target organ. For example, SNS activity shortens inter beat intervals (IBI) of the heart whereas PNS activity extends the IBIs. Autonomic control is typically reciprocal but co-activation of SNS and PNS or independent activation of one branch can also happen.

The status of the ANS reflects how stressed/recovered a person is. The status of the ANS is a crucial parameter when determining if the person has had significant amount of mental or physical load and when determining how well the person is able to recovery from the load. In a daily life, the person is exposed to many stressors that may include, for example a self-inflected stressor such as a physical workout or an unpredictable mental stressor such as traffic jam. Stress response is a non-specific arousal, both physical and mental, to situations or events (i.e. stressors) that the person perceives as threatening or challenging. The stress response is characterized by a decrease in PNS activity and an increase in SNS activity as well as a release of stress hormones including adrenaline, noradrenaline and cortisol. If the person encounters frequent stressors, even minor ones, the load accumulates and the stress response (i.e. physical and mental arousal) is sustained even though the stressor itself has passed. A balance between SNS and PNS activity is shifted towards SNS dominance even at night when the person sleeps when no special demands are made on the person. This sustained arousal reflects incomplete recovery over the course of the day. Moreover, if the status of the ANS remains disturbed night after night, it reflects accumulated load and insufficient recovery at day and night time. In this situation, the person has to mobilize more effort to cope with challenges he face, for example in sports or at work. This may cause performance/health impairment in the long run. In general, measurement of the ANS function does not only indicate stress/recovery but also provide information of cardiovascular health that may be risked due to a prolonged stress.

A publication by Celka et al (15.6.2019) "Pulse wave harmony: ancient wisdoms for modern age" at URL: https:/ /www.researchgate.net discloses use of photoplethysmography combined with traditional Tibetan Pulse reading for estimation of energies of a person: activity, transformation and stability. The publication teaches analysis that gives insights to dynamics of pulse waves under stress and relaxation.

WO 2017/212120 discloses a solution for estimating blood pulse wave characteristics by using multiple measurement locations of a human body. According to an aspect, a method comprises: detecting, in a first measurement signal measured by a first heart activity sensor associated with a first location of a human body, a first occurrence of a blood pulse wave; detecting, in a second measurement signal measured by a second heart activity sensor from a second location of the human body different from the first location, a second occurrence of the blood pulse wave; estimating, on the basis of said detections synchronized to a common clock, time characteristics of the blood pulse wave; and computing, on the basis of said time characteristics, a metric representing a physiological condition of the human body.

US 2007/056582 discloses methods and devices for evaluating and treating stress and thereby disorders caused or exacerbated by stress. More particularly methods and devices for identifying RSA waves during respiration which provide a subject with real-time RSA wave information are provided. These methods and devices also can be used to identify drop points in RSA waves. Such methods and devices provide subjects with the ability to maintain parasympathetic outflow and thereby prevent and/or reduce levels of stress.

US 2015/282723 discloses a device that detects the pulse rate and therefrom additionally determines the heart rate variability. In addition, at least one parameter related to the history of one of the two above mentioned values shall be used. Preferably, the deviation of the cardiac frequency and of the heart rate variability from a reference value is integrated and used as an additional stress indicator.

JP 2013/128659 discloses a portable respiration induction apparatus that includes an apparatus body including: a detection part detecting detection signals from the fingers of the user; a heartbeat calculating part calculating the heartbeat variation curve from the detection signals detected by the detection part; a storage part storing a guiding respiration rhythm; and a display part displaying the heartbeat variation curve calculated by the heartbeat calculating part, and the guiding respiration rhythm stored in the storage part. The apparatus body comprises an upper-side body and a lower-side body which are turnably pivoted, and allows the finger of the user to be placed between the upper-side body and the lower-side body.

WO 2009/104127 discloses a system and a kit for stress and relaxation management. A cardiac activity sensor is used for measuring the heart rate variability signal of the user and a respiration sensor for measuring the respiratory signal of the user. The system contains a user interaction device having an input unit for receiving user specific data and an output unit for providing information output to the user. A processor is used to assess the stress level of the user by determining a user related stress index. The processor is also used to monitor the user during a relaxation exercise by means of determining a relaxation index based on the measured HRV and respiratory signals, the relaxation index being continuously adapted to the incoming measured signals and based thereon the processor instructs the output unit to provide the user with biofeedback and support messages. Finally, the processor uses the user specific data as an input in generating a first set of rules defining an improvement plan for self -management of stress and relaxation. The first set of rules is adapted to trigger commands instructing the output unit to provide the user with motivation related messages. Also, at least a portion of said user specific data is further used to define a second set of rules indicating the user' s personal goals.

WO 2019/077304 discloses a device for guiding breathing of a user, the device comprising circuitry configured: to output a breathing signal for guiding the user to breathe in and breathe out during a breathing cycle defined over a time period; to receive a first input signal indicative of a first physiological property of the user, wherein a heartbeat of the user is recognisable based on the first input signal; based on the first input signal, to determine, at the end of a first breathing cycle, if there is a negative rate of change in the heartbeat interval of the user and, if there is negative a rate of change in the heartbeat interval of the user, to control the breathing signal to increase the time period of the breathing cycle during which the user is guided to breathe in and breathe out; to receive a second input signal indicative of a second physiological property of the user, wherein a physiological response of the user to a perturbation of the cardiovascular system of the user during the breathing cycle is recognisable based on the second input signal; based on the second input signal, to determine, at the end of a second breathing cycle subsequent to the first breathing cycle, if the physiological response of the user to the perturbation of the cardiovascular system of the user during the second breathing cycle is less than a predetermined threshold and, if the physiological response of the user to the perturbation of the cardiovascular system of the user during the second breathing cycle is less than the predetermined threshold, to control the breathing signal to reduce the time period of the breathing cycle during which the user is guided to breathe in and breathe out.

### BRIEF DESCRIPTION

The invention is defined by the independent claims. Embodiments are defined in the dependent claims.

### BRIEF DESCRIPTION OF THE DRAWINGS

In the following the invention will be described in greater detail by means of preferred embodiments with reference to the accompanying drawings, in which
Figure 1 is illustrates a measurement system usable in embodiments of the invention;
Figures 2A and 2B illustrate processes for estimating a stress level of a human according to some embodiments of the invention;
Figures 3A to 3C illustrate different distributions of inter-heartbeat-interval samples in a frequency domain;
Figure 4 illustrates an embodiment for using respiratory frequency as a reference frequency in the process of Figure 2;
Figure 5 illustrates a procedure for controlling user's respiratory frequency while measuring heart activity according to an embodiment of the invention;
Figure 6 illustrates an embodiment using two different algorithms for estimating the stress level and computing an aggregate stress level;
Figure 7 illustrates an embodiment for determining one or more stressors influencing the user while measuring heart activity measurement data for stress level estimation;
Figure 8 illustrates an embodiment of an indicator indicating a momentary stress level;
Figure 9 illustrates a block diagram of an apparatus according to an embodiment of the invention; and
Figure 10 illustrates a series of display views forming an embodiment of breathing instructions.

### DETAILED DESCRIPTION OF EMBODIMENTS

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

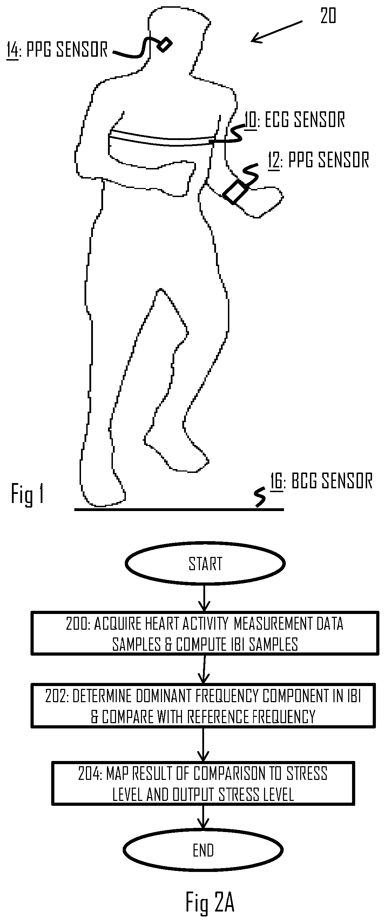

Figure 1 illustrates a measurement system comprising sensor devices that may be used in the context of some embodiments of the present invention. The sensors may employ one or more measurement technologies for measuring heart activity of a user 20. For example, at least one sensor device 10 may be configured to measure electrocardiogram (ECG) of the user 20. Such an ECG sensor 10 may comprise one or more electrodes arranged to be in contact with the user's 20 skin in order to measure electric charges generated during each heartbeat. The ECG sensor may be portable to enable the measurement during an outdoors physical exercise, such as running or cycling.

At least one sensor device 12, 14 may be configured to measure a photoplethysmogram (PPG) optically. PPG represents a volumetric measurement of an organ. A PPG sensor 12, 14 may comprise a light source such as a light emitting diode (LED) configured to illuminate a skin of the user 20 and, further, comprise a light-sensitive sensor such as a photodiode configured to measure changes in light reflected from the illuminated skin. With each cardiac cycle, the heart pumps blood to peripherial arteries. Even though this blood wave pulse is damped by the artery system as it propagates, it is enough to distend arteries and arterioles in the subcutaneous tissue. If the light source and the light-sensitive sensor are place appropriately against the skin, the blood wave pulse can be detected as a change in the reflecting light measured by using the light-sensitive sensor. Each cardiac cycle appears as a peak in a measurement signal acquired through the light-sensitive sensor. The blood pulse wave may be modulated by multiple other physiological systems and, therefore, the PPG may also be used to monitor breathing, hypovolemia, and other physiological conditions. The PPG may be measured at various locations of the human body, e.g. from a wrist (sensor 12), head, ear canal or ear leaf (sensor 14).

At least one sensor device 16 may be configured to measure a ballistocardiogram (BCG). The BCG is a measure of ballistic forces generated during the heartbeat. Ballistocardiogram characterizes motion of the human body resulting from the ejection of blood into the great vessels during each heartbeat. The BCG shows on a frequency range between 1 and 20 Hertz (Hz), and is caused by the mechanical movement of the heart. As the ECG and the PPG, the BCG can be recorded by using a non-invasive sensor 16 from the surface of the body. One The BCG sensor 16 may be a ballistocardiographic scale configured to measure a recoil of the human body standing on the scale. The recoil is caused by the heartbeat and can be measured from the user standing on the BCG scale, e.g. by using a pressure sensor. The BCG scale may be configured to show the user's 20 heart rate as well as weight.

As described above, the status of the ANS reflects on how stressed the user 20 is, and the functions of the ANS affect the cardiovascular system as well. This means that, by using proper measurements and signal processing, a stress level of the user may be determined from cardiovascular measurement data or, in other words, heart activity measurement data. Figure 2A illustrates a flow diagram of a method for estimating the stress level from the heart activity measurement data. Referring to Figure 2A, the method comprises as performed by an apparatus: acquiring (block 200) a set of heart activity measurement data samples measured by a heart activity sensor from the user; computing (block 200) a set of inter-heartbeat interval (IBI) samples of the set of heart activity measurement data samples; determining (block 202) a dominant frequency component from the set of inter-heartbeat interval samples and comparing the determined dominant frequency component with a reference frequency; mapping (block 204) a result of said comparison to a stress level of the user according to a determined mapping criterion; and outputting (block 204) the stress level through an interface of the apparatus.

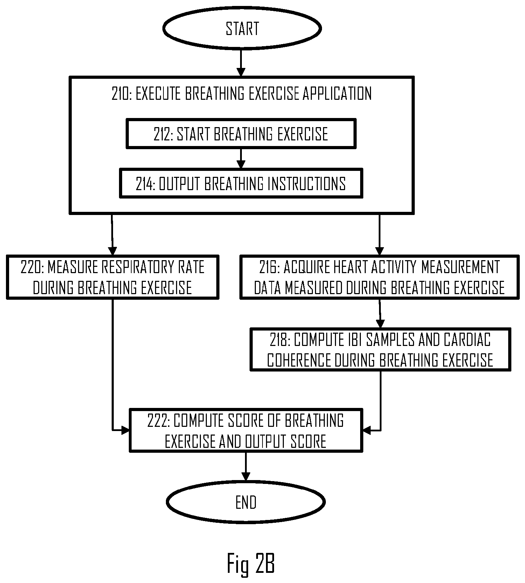

It may be beneficial to carry out the stress level estimation in a situation where conditions for measuring the stress level are controlled. Figure 2B illustrates such an embodiment of a process for estimating the stress level of the user. Referring to Figure 2B, the process comprises as performed by the apparatus: executing (block 210) a breathing exercise application by the apparatus, starting (block 212) a breathing exercise of the breathing exercise application and outputting (block 214), during the breathing exercise, breathing instructions to a user of the apparatus; acquiring (block 216) a set of heart activity measurement data samples measured by a heart activity sensor from the user during the breathing exercise; computing (block 218) a set of inter-heartbeat interval samples of the set of heart activity measurement data samples; computing (block 218) a cardiac coherence of the user during the exercise from the set of inter-heartbeat interval samples; measuring (block 220) a respiratory rate of the user during the breathing exercise; computing (block 222) a score of the breathing exercise on the basis of the respiratory rate and the cardiac coherence, the score indicating a stress level of the user; and outputting (block 222) the score through an interface of the apparatus.

The set of heart activity measurement data samples may be measured by using any one or more of the above-described heart activity sensors 10 to 16.

The apparatus may be any one of the above-described sensors 10 to 16, a personal electronic devices such as a portable computer, wrist-worn computer, or a mobile phone or a tablet computer, or the apparatus may be a server computer.

In an embodiment, the interface is a user interface of the apparatus, e.g. a display screen. In another embodiment, the interface is a network adapter such as a wireless or wired communication circuitry, e.g. a Bluetooth^{®} interface complying with the Bluetooth^{®} standard specifications.

The IBI samples characterize a time interval between consecutive heartbeats detected from the heart activity measurement data. The computation of an IBI sample may comprise detecting a determined heartbeat feature from the heart activity measurement data, e.g. a peak in ECG or PPG measurement data, and measuring a time interval between consecutively detected heartbeat features. The IBI samples also characterize heart rate variability (HRV).

In an embodiment of the process of Figure 2A, the dominant frequency component may be computed from the IBI samples by converting the IBI sample set into a frequency domain by using a Fourier transform, for example, and computing a frequency sample having the highest value. In another embodiment, the dominant frequency component is computed in a time domain by employing a filter bank, for example, inputting the IBI samples to the filter bank and measuring a signal level at an output of each filter of the filter bank. The filter bank comprises multiple filters tuned to different frequency bands. A frequency of a filter bank outputting the highest signal level may be determined as the dominant frequency component. The skilled person may, however, employ other signal processing means in determining the highest frequency component.

The distance between the dominant frequency component in the IBI sample set and the reference frequency represents the stress level. In an embodiment, the closer the dominant frequency component is to the reference frequency, the lower the stress level is. Accordingly, the more distant the dominant frequency component is from the reference frequency, the higher is the determined stress level. Block 204 may employ a mapping table or a mapping rule that maps each distance to one of a plurality of stress levels by using this principle: the higher distance between the dominant frequency component and the reference frequency is associated with a higher stress level.

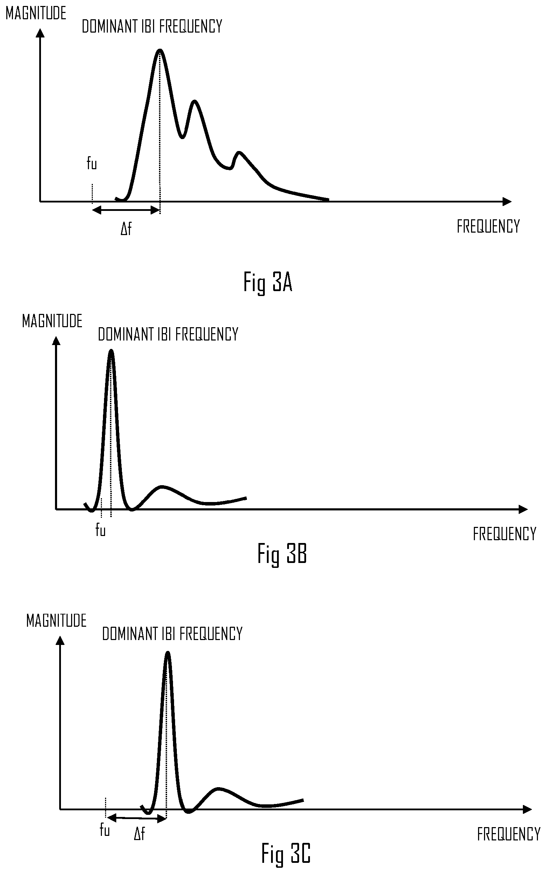

Figures 3A, 3B, and 3C illustrate different IBI sample sets. The reference frequency is represented by *f_{µ}* and the distance between the dominant frequency component and the reference frequency *f_{µ}* is represented by Δ*f.* In Figure 3A, the IBI sample set has a high deviation in the frequency domain while in Figures 3B and 3C the deviation is low. In Figure 3B, the dominant frequency component (dominant IBI frequency) is close to the reference frequency while in Figures 3A and 3C the dominant reference frequency is more distant to the reference frequency. Figure 3B thus illustrates a situation where the user is determined to have a low stress level while Figures 3A and 3C illustrate situations where the user is determined to have a higher stress level.

The deviation of the IBI samples in the frequency domain represents the cardiac coherence of the user 20. Figure 3A illustrates poor cardiac coherence with the dispersed IBI sample set while Figures 3B and 3C illustrate higher cardiac coherence with the concentrated IBI sample set. The cardiac coherence also represents the stress level of the user. A higher cardiac coherence is associated with the lower stress level, while a lower cardiac coherence is associated with the higher stress level. In an embodiment of Figure 2A, the cardiac coherence is computed and used as additional information for determining the stress level in block 204. Accordingly, a combination of the computed cardiac coherence and the distance between the dominant frequency component and the reference frequency is mapped to the stress level according to a determined mapping rule. In the embodiment of Figure 2B, the cardiac coherence may be computed in block 218 according to any one of the embodiments using the proximity of the IBI samples to the dominant frequency component.

Referring to Figures 3A to 3C, the samples of Figure 3B may be mapped to the lowest stress level because of high cardiac coherence and close proximity of the dominant frequency component to the reference frequency. The samples of Fig-ure 3A may be mapped to the highest stress level because of low cardiac coherence and high distance between the dominant frequency component and the reference frequency. The samples of Figure 3C may be mapped to a stress level between the lowest and the highest stress levels because of high cardiac coherence but high distance between the dominant frequency component and the reference frequency.

In an embodiment, the cardiac coherence is defined in terms of deviation or variance of the IBI sample set. High deviation or high variance is associated with low cardiac coherence while low deviation or low variance is associated with high cardiac coherence.

The cardiac coherence may be understood to represent a degree of a harmonic component in a signal represented by the set of IBI samples, higher degree of the harmonic component indicating higher cardiac coherence. In other words, the cardiac coherence represents how sinusoidal the signal represented by the IBI sample is. The IBI samples have a correlation with the respiratory rate in the sense that the IBIs shorten when the inhales and lengthen during exhalation, thus forming a periodic characteristic. The purer the periodicity, the higher the cardiac coherence. The signal represented by the IBI samples is less correlated, i.e. more noise-like, during normal breathing and more correlated, i.e. more harmonic, during deep breathing. Therefore, a more accurate estimate of the stress level may be acquired when the user is breathing deeply. Therefore, the breathing instructions may instruct the user to breathe deeply, e.g. four to six breaths per minute.

In an embodiment, the cardiac coherence is estimated through correlation with a sinusoidal reference signal. The reference signal may be aligned with the breathing instructions such that the reference signal represents a sinusoidal signal that matches with the instructed respiratory frequency. The correlation between the reference signal and the IBI samples may then directly represent the cardiac coherence. Other embodiments for computing the cardiac coherence from the IBI samples are equally possible.

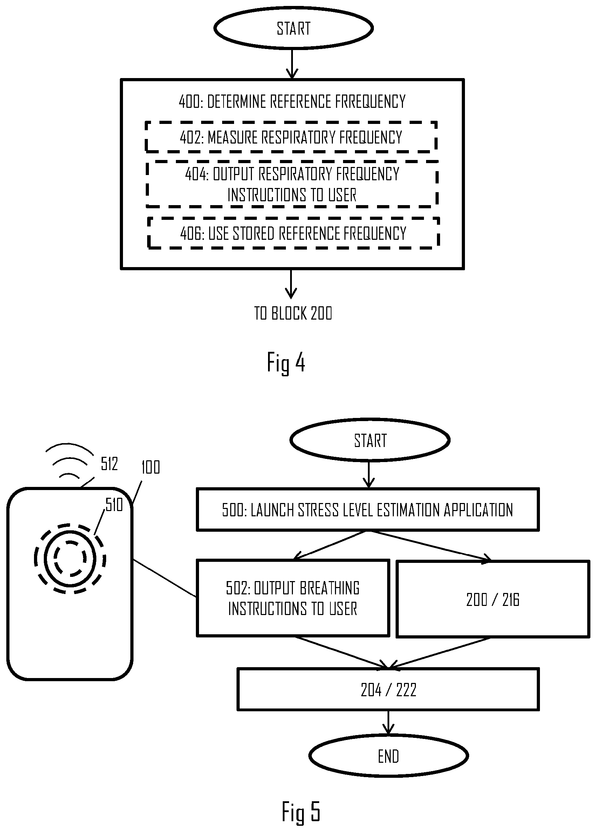

In an embodiment, the reference frequency represents a respiratory frequency or a breathing frequency of the user at the time of measuring the heart activity measurement samples. Referring to Figure 4 illustrating an embodiment of Figure 2, the method further comprises determining (block 400) a respiratory frequency of the user 20 at the time of measuring the heart activity measurement samples and using the respiratory frequency as the reference frequency.

In an embodiment, the reference frequency is lower than one Hertz (Hz) or even lower than 0.5 Hertz.

In an embodiment of Figure 4, the respiratory frequency is stored beforehand and used as a default value in block 406. In this embodiment, the reference frequency may be determined from user parameters input by the user. The used user parameters may include one or more of the following: age, gender, weight, and body composition. For example, the respiratory frequency increases with the age from a child to adult age and lower with aging during the adulthood. Average respiratory frequency for adults is 0.2 Hz. Obesity has long been recognized as having significant effects on respiratory function. Obese persons tend to have higher respiratory rates and lower tidal volumes. Total respiratory system compliance is reduced for a variety of reasons. Lung volumes tend to decrease, especially expiratory reserve volume. Spirometry, gas exchange and airway resistance all tend to be relatively well preserved when adjusted for lung volumes. Weight loss leads to a reversal of these changes. For all of these changes, the distribution of fat, that is, upper versus lower body, may be more important than body mass index. The weight and body composition thus may be used as inputs to the determination of the reference frequency. In the embodiment using block 406, the reference frequency may be based on the user parameters and study of a population. Optimal reference frequencies may be determined for each age, gender, weight, and body composition by using the population study, and the reference frequency may thus be determined for the user's parameters.

In an embodiment, the user is instructed to breathe with a determined respiratory frequency forming the reference frequency (block 404). Block 404 may be executed in the process of Figure 2B, for example. The breathing instructions may be output to the user through the user interface while performing the heart activity measurements. In this manner, it may be controlled that the user is performing the measurements under suitable conditions and that the estimated stress level is accurate. It may be desired that the measurements are carried out under static conditions, e.g. under a steady respiratory frequency. Accordingly, the user may be instructed to sit or lie still for the duration of the measurements.

In an embodiment, the respiratory frequency is input by the user.

In an embodiment, the respiratory frequency is measured (block 402) while performing the heart activity measurements. The respiratory frequency may be measured by using an accelerometer or a strain gauge attached to the user. There also exist solutions for estimating the respiratory frequency or respiratory rate from the ECG or PPG measurement data, for example. The reference frequency may then be computed from the measured respiratory frequency, e.g. by using an average function. In an embodiment, block 402 is employed when the user 20 is sleeping. Modern wearable activity monitoring devices and training computer are capable of detecting determining when the user is sleeping. Such a detection may trigger an apparatus to execute the process of Figure 2 by using block 402 for determining the reference frequency.

The respiratory rate may be understood as equal to the respiratory frequency.

Performing the stress level estimation when the user is asleep provides for a long-term estimation of the stress level and comparable results that span over several days or weeks. Let us for the simplicity sake that the user sleeps at nights. The status of ANS and the stress level may be based on comparing the measurement of each night to a baseline value of the user. The baseline value may be an average value of the stress level from a period of days when the user's status is considered to be normal or stress level low. Measured motion data and an activity monitoring algorithm may be employed to ignore days with exceptional physical activity. Similarly, the measured motion data and a sleep quality estimation algorithm may be employed to exclude nights with disturbed sleep or an exceptional sleep-wake rhythm. If the user provides other information, such as days when the user is ill, such information may be used to ignore days when the ANS status is not reflecting the normal baseline of the user.

When the individual stress level measurement is compared with the baseline, a limit may be designed to define whether or not the difference between the current measurement and the baseline is significant.

In an embodiment, a stress level estimation application is stored as a computer program product in the apparatus. The stress level estimation application may be considered as a measurement function in a fitness application of the apparatus. The breathing exercise application executed in the embodiment of Fig-ure 2B is an example of the stress level estimation application.

Figure 5 illustrates operation of the apparatus when executing the stress level estimation application. Referring to Figure 5, the stress level estimation application is launched in block 500 as a response to a user input, for example. During the execution of the application, the measurement of the heart activity measurement data samples (e.g. block 200) and the output of the breathing instructions (block 502) are performed simultaneously by the apparatus. As illustrated in Fig-ure 5, the instructions of the respiratory frequency may be output as a visual output and/or as an audio output. Regarding the visual output, an object 510 changing a shape in a periodic manner may be displayed on a display screen of the apparatus 100. The periodicity of the shapeshifting may be the respiratory frequency. Regarding the audio output, a periodic audio signal may be output through a loudspeaker 512 of the apparatus 100. The periodicity of the audio signal may be in an audio volume or audio frequency.

In an embodiment, the animation of the object 510 is periodic and adapted to the instructed breathing frequency in the following manner. The animation may follow the same trajectory regardless of the instructed breathing frequency but the speed of the animation may be adapted to the instructed breathing frequency. Accordingly, speed of a motion vector of the object 510 during the animation may be proportional to the instructed breathing frequency: the higher the breathing frequency the higher the speed. Figure 5 illustrates a circle that grows and shrinks to illustrate the breathing frequency. Assuming that the circle grows as an illustration of inhalation and shrinks as an illustration of exhalation, the speed vector of a point (a pixel) on the circle is proportional to the instructed breathing frequency. During inhalation, the speed vector points away from the centre of the circle and has a magnitude proportional to the instructed breathing frequency. Analogously during the exhalation, the speed vector points to the centre of the circle and has a same magnitude proportional to the instructed breathing frequency. A corresponding adjustment of the animation speed to match with the respiratory rate can be applied to other animations as well.

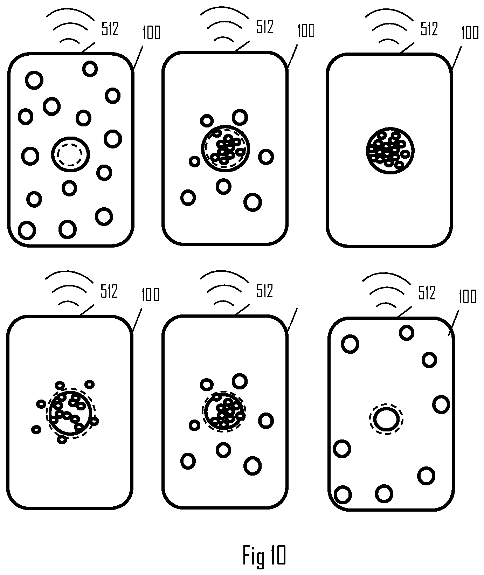

In another embodiment, another illustration is displayed. The illustration may be such that it enables the user to see or evaluate a remaining inhale/exhale time of a current inhale/exhale period. This helps the user to find breathing rhythm. An example of such an illustration is provided in Figure 10, wherein a circle or a "container" is illustrated in the middle of a display screen and air bubbles or similar objects are controlled to travel to the container from one or more edges of the display screen in an inhale period. The container may also expand during the inhale period, as illustrated in top three display views of Figure 10. The inhale period ends when all the objects are inside the container (see top-rightmost display view in Figure 10). In the exhale period illustrated by the lower three display views of Figure 10, the objects are controlled to exit the container and travel towards the edge(s) of the display screen, and the exhale period ends when all the objects have disappeared from the display screen (shortly after the bottom-rightmost display view in Figure 10). The container may shrink during the exhale period. Other similar animations can readily be foreseen.

In an embodiment, the breathing frequency is defined in terms of an inhale time and an exhale time. The inhale time may equal to the exhale time but, in some embodiments, the inhale time may differ from the exhale time. In an embodiment, the exhale time is (always) greater than or equal to the inhale time. The inhale time and the exhale time may be preset and determined by the application but, in some embodiments, the user may change the inhale time and/or exhale time before starting the breathing exercise. The preset value may be five seconds for inhale time and five seconds for exhale time, for example. The application may prevent the user from setting an inhale time that is greater than the exhale time. The user may set the duration of the breathing exercise.

Block 502 and associated measurements may be performed for a determined duration or until a sufficient number of heart activity measurement data samples have been measured. The computation of the stress level (block 204) or the score for the breathing exercise (block 222) may then be carried out after measuring the set of heart activity measurement data samples. Regarding block 200, the heart activity measurement samples may be measured and acquired simultaneously with block 502 and the IBI samples may be computed after the set of heart activity measurement samples have been measured.

An alternative input triggering block 500 may be from an activity monitoring application of the apparatus, reporting that the user has been detected to fall asleep. In such a case, block 502 may be omitted. The apparatus may be configured to determine the input triggering block 500 and, as a result either execute block 502 or omit block 502 on the basis of the input. The user input may trigger execution of block 502 while the activity monitoring input may cause omission of block 502.

In an embodiment, upon detecting that the score or the estimated stress level exceeds a determined threshold THₐₗₐᵣₘ, the apparatus may trigger output of one or more instructions instructing the user to perform a mental and/or physical exercise that reduces the stress level, or output of a proposal for performing such an exercise.

The score for the breathing exercise may be computed at least after the breathing exercise has ended. In another embodiment, the score is computed during the breathing exercise as a momentary score and output as a feedback to the user so that the user may evaluate the performance during the exercise. The score may be computed by using only a subset of the measurement data available for the computation of the cardiac coherence and the respiratory rate, e.g. a certain amount of latest measurement data measured defined by a time window. Accordingly, when the exercise starts the apparatus may first gather a sufficient amount of measurement data to compute the first momentary score and output the first momentary score. As the exercise continues, the measurement data acquired at the beginning of the exercise may be omitted from the computation of later momentary scores. When the exercise has ended, all or at least a majority of the measurement data acquired during the exercise may be used in the computation of a total score for the exercise.

A blood pulse is modulated on its way from the heart and through the human body. The modulation may be caused by various physiological conditions and functions. Therefore, characteristics of the blood pulse wave may comprise representation of such physiological conditions. One set of such characteristics may include propagation characteristics of the blood pulse wave. The propagation characteristics may be considered as time characteristics that represent a pulse transit time (PTT), for example, within a certain distance in the human arteries. Equivalent characteristics may include pulse wave velocity (PWV) which is proportional to the pulse propagation time and, therefore, can be considered to represent the time characteristics of the blood pulse wave.

The PWV is mainly a function of arterial stiffness, arterial blood pressure, the heart rate, the age, and conditions of the arteries (affected by smoking habits, arteriosclerosis, high blood pressure, etc.). Arterial stiffness is modified during mental or physical stress due to local SNS activity. For an otherwise healthy person, the PWV or its corresponding PTT can thus be a measure of stress if the measure is performed when the blood pressure is at its lowest value, e.g. when lying still or before breakfast. The PWV can be estimated by different means such as: 1) using a reference signal such as the ECG R-wave together with a distal measure of the blood pressure such as for example measured by the PPG placed on a specific body location that can sense the blood pressure wave and influences of vascular tone, e.g. on a wrist, finger, or ear; 2) from the sole features of the PPG by using two spatially separated PPG measurement points and detection of the same blood pulse wave at the two measurement points. The PWV may be measured on the basis of a time of occurrence of the detection of the blood pulse wave at each measurement point and distances from the heart to each measurement point. As an alternative to the PPG, arterial applanation tonometery (ATO) or Doppler Ultrasound flow meter may be employed.

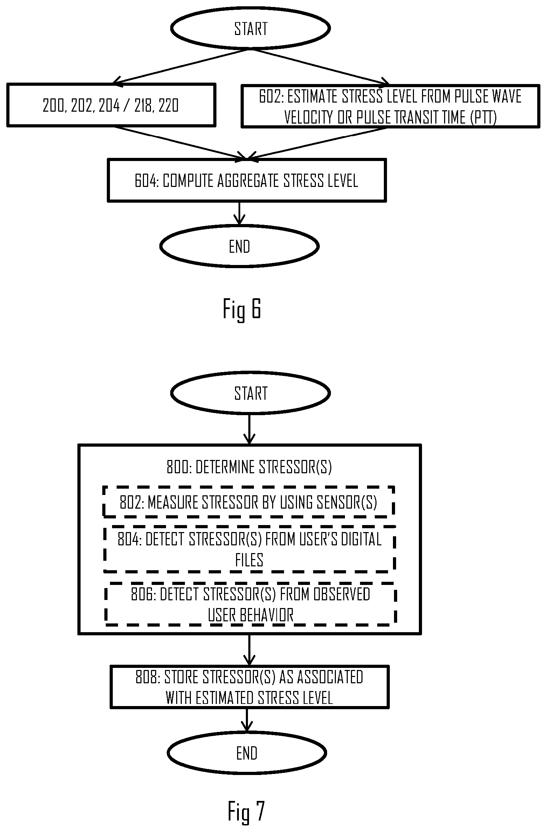

An embodiment estimates the stress level by using the method of Figure 2 or any one of its embodiments and, further, a second method where a metric associated with the PWV is measured from the user and is mapped to the stress level. Figure 6 illustrates such an embodiment. Referring to Figure 6, upon triggering the estimation of the stress level, the apparatus may perform the embodiment of Fig-ure 2A or 2B and acquire a first stress level estimate, e.g. the score of Figure 2B. Meanwhile, the apparatus may estimate the stress level from the PWV or PTT in block 602. The measured PWV or PTT may be mapped to a stress level according to a determined mapping scheme, thus acquiring a second stress level estimate. In block 604, the apparatus determines an aggregate stress level by using the first stress level estimate and the second stress level estimate.

In an embodiment combining embodiments of Figure 2B and 6, the user may define in settings of the breathing exercise application whether or not to incorporate the PWV or PTT measurement into the computation of the score for the breathing exercise. In another embodiment, the breathing exercise application may automatically set the PWV/PTT measurement to be included in the breathing exercise, e.g. it may be determined that the PWV/PTT measurement shall be included once a week or with another periodicity.

In an embodiment, block 604 comprises: estimating an accuracy of the first stress level estimate Q₁ and an accuracy of the second stress level estimate Q₂ and comparing each accuracy Q₁, Q₂ with a determined accuracy threshold TH₁, TH₂; and selecting, on the basis of the threshold comparison, one of the stress level estimates or a combination of both stress level estimates as the aggregate stress level Qₜₒₜ. The combination may include unequal weighting of the stress level estimates. The weights may be based on, for example, the estimated accuracy of each estimate: a higher weight may be assigned to a more accurate estimate.

A stressor is a chemical or biological agent, environmental condition, external stimulus, or an event that causes stress to the user. An event that triggers the stress response may include environmental stressors (hypo or hyper-thermic temperatures, elevated sound levels, over-illumination, overcrowding), daily stress events (e.g., traffic, lost keys, quality and quantity of physical activity), life changes (e.g., divorce, bereavement), workplace stressors (e.g., high job demand vs. low job control, repeated or sustained exertions, forceful exertions, extreme postures), chemical stressors (e.g., tobacco, alcohol, drugs), or social stressors (e.g., societal and family demands).

In an embodiment, the process of Figure 2A, 2B, or any one of the above-described embodiments comprises: detecting at least one stressor during measurement of said heart activity measurement samples; and storing information on the detected at least one stressor as associated with the estimated stress level. Fig-ure 7 illustrates such an embodiment where the stressor(s) are detected or determined in block 800 and stored as associated with the estimated stress level in block 808. Block 800 may comprises one or more of the sub-blocks 802, 804, 806.

In an embodiment, the detection of a stressor is based on measurements (block 802). For example, the daily activity of the user may be measured and, if the activity exceeds a determined activity threshold, the detection of a stressor may be triggered. On the other, if the activity deviates from a normal or average daily activity by a determined amount, the detection of the stressor may be triggered. As another example, the location of the user may be measured. If the user is distant from a home location, e.g. in a different country, the detection of the stressor may be triggered. If the user's location is associated with an abnormal event, e.g. a football stadium or a night club, the detection of the stressor may be triggered. If the user's location is at work longer than a conventional work day for extended period of time, the detection of the stressor may be triggered. As yet another example, an amount of light during the day and/or night may be measured. If a total amount of light is below a threshold or amount light during night is above a threshold, the detection of the stressor may be triggered. As yet another example, an ambient audio noise level may be measured. If the noise level is above a threshold, the detection of the stressor may be triggered. User's voice may indicate the stress level. Accordingly, a voice analysis algorithm may be employed to determine whether or not the user is under stress. If stress is detected in the voice analysis, the detection of the stressor may be triggered. As yet another example, meal times may be detected on the basis of motion data. Irregular meal times may cause detection of a stressor.

In another embodiment, the detection of a stressor is based on analysing user's digital files such as events stored in a digital calendar or posts in social media (block 804). If the analysis indicates abnormal events or posts, the detection of the stressor may be triggered. Detection may be based on a number of calendar events exceeding a threshold (too busy) or lack of calendar events. The type of calendar events may also be evaluated. For example, too heavy training schedule, no training schedule, or too many sports competitions may cause detection of a stressor.

As described in connection with blocks 802 and 804, the user's behaviour may be monitored and compared with a normal daily behaviour (block 806). When the user deviates from the normal daily behaviour, the detection of the stressor may be triggered. If the stress level estimation is carried out under an influence of one or more stressors, the presence of the stressors stored in association with the stress level estimation may help in reducing the stress.

In an embodiment, detection of one or a plurality of stressors in the process of Figure 2A, 2B, or when measuring the user's daily activity, location, etc. may trigger scheduling of at least one additional breathing exercise to the user's schedule, e.g. calendar. Alternatively, or additionally, a proposal or an instruction to conduct a breathing exercise may be output to the user through the user interface as a response to the detection. The user may be provided with an option to respond to the proposal by providing a one-action input that directly triggers execution of the breathing exercise. The one-action input may be a one-click input through a user input device such as a button or touch of an icon displayed together with the proposal on a touch-sensitive display. Each stressor may be given a weight or a score, and a current total score of the detected stressors may be compared with a threshold. Upon detecting the total score above the threshold, the scheduling or proposal of the breathing exercise may be triggered.

In an embodiment, a plurality of zones are provided for the score. Each zone may be defined by a unique range of values of the score. A higher score may indicate a better performance and a lower stress level of the user. The apparatus may output, through the interface during the breathing exercise and/or after the exercise, the score by utilizing the zones. In the embodiments where the momentary score is computed during the exercise, the apparatus may accumulate a duration the score stays within each zone during the breathing exercise. After the exercise, the apparatus may output the score in the form of the duration the score has spent in each zone, e.g. in the following form:

| **Zone** | **Time** |
|---|---|
| Zone 1 (A - B) | 0:27 |
| Zone 2 (B - C) | 1:34 |
| Zone 3 (C - D) | 0:29 |
| Total | 3:00 minutes |

A, B, C, and D may have arbitrary values defining the scale for the score.

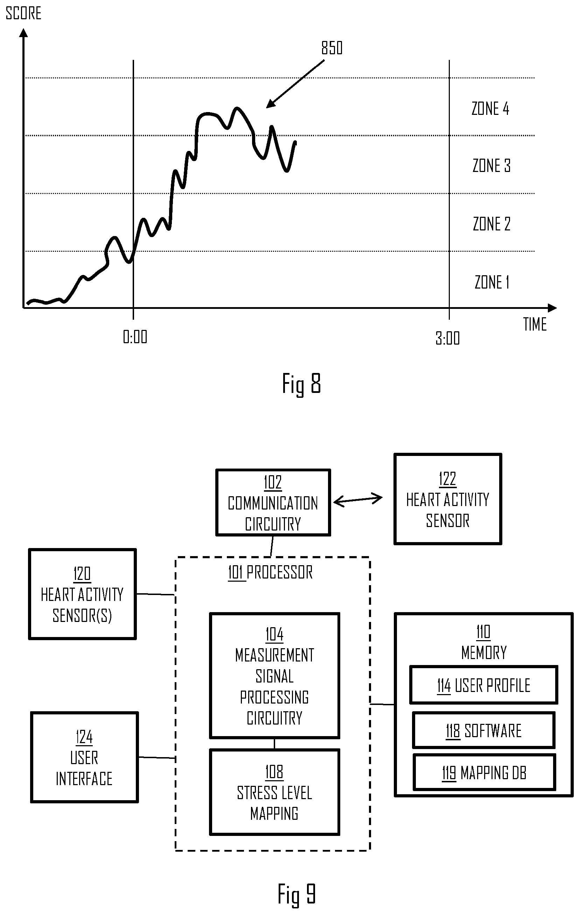

In an embodiment, the apparatus outputs, during the breathing exercise an indicator indicating a zone where the momentary score currently resides as a feedback to the user. Figure 8 illustrates an embodiment where the apparatus draws and outputs a graph 850 indicating the progress of the score during the breathing exercise. '0:00' indicates a start time of the exercise. When the graph is illustrated to the user during the exercise, the user can quickly see the progress of the exercise and the zone where the momentary score currently resides. When the exercise has ended the graph is complete up to the end time of the exercise, e.g. 3:00 in Figure 8.

Figure 9 illustrates an embodiment of an apparatus configured to carry out at least some of the above-described functions in estimating the stress level of the user 20. The apparatus may comprise an electronic device comprising at least one processor 101 and at least one memory 110. The processor 101 may form or be a part of a processing circuitry. The apparatus may further comprise a user interface 124 comprising a display screen or another display unit, an input device such as one or more buttons and/or a touch-sensitive surface, and an audio output device such as a loudspeaker. In some embodiments, the user interface 124 comprises a haptic output device configured to provide haptic indications to the user 20.

The processor 101 may comprise a measurement signal processing circuitry 104 configured to acquire the heart activity measurement data and to process the heart activity measurement data samples. In an embodiment, the measurement signal processing circuitry 104 is configured to compute the IBI samples and to estimate the stress level from the IBI samples, as described above. The measurement signal processing circuitry may further compute the cardiac coherence and the respiratory rate from the heart activity measurement data samples and, optionally, from other measurement data. The measurement signal processing circuitry 104 may comprise a time characteristics estimation circuitry configured to estimate the time characteristics such as the PTT or PWV from received detected measurement signals, as described above in the embodiment of Figure 5. The measurement signal processing circuitry 104 may output the dominant frequency of the IBI samples, the respiratory rate, the cardiac coherence, the computed time characteristics, or a subset of these parameters, to a stress level mapping circuitry 108 configured to map the received information to a stress level according to any one of the above-described embodiments. The memory 110 may store a user profile 114 storing the reference frequency for the user. The memory may further store a mapping database 119 defining mapping rules for determining the stress level from the time characteristics, the distance between the dominant frequency components and the reference frequency, the cardiac coherence, and/or the respiratory rate with respect to the instructed breathing.

Upon successful computation of the stress level, the processor 101 may output an indication about the measured stress level to the user via the user interface 124. The indicator may be a display indicator displayed on the display unit of the user interface 124, an audio output, or a haptic output.

The apparatus may comprise a communication circuitry 102 connected to the processor 101. The communication circuitry may comprise hardware and software suitable for supporting Bluetooth^{®} communication protocol such as Bluetooth Smart specifications. It should be appreciated that other communication protocols are equivalent solutions. The processor 101 may use the communication circuitry 102 to transmit and receive frames according to the supported wireless communication protocol. In some embodiments, the processor 101 may use the communication circuitry 102 to transmit the heart activity measurement data and/or estimated stress levels to another apparatus, e.g. to a cloud server storing the user's 20 user account.

In an embodiment, the apparatus comprises at least one heart activity sensor 120. The heart activity sensor(s) 120 may comprise one or more of the above-described sensors such as an ECG sensor 10, PPG sensor 12, 14, and the BCG sensor 16. In another embodiment, the apparatus may communicate with at least one heart activity sensor 122 through the communication circuitry 102. The at least one heart activity sensor 122 may comprise an external heart activity sensor with respect to the apparatus. The heart activity measurement data may thus be acquired from an internal or external heart activity sensor for the estimation of the stress level.

It will be obvious to a person skilled in the art that, as the technology advances, the inventive concept can be implemented in various ways. The invention and its embodiments are not limited to the examples described above but may vary within the scope of the claims.

## Claims

1. An apparatus comprising means for performing the following:
executing a breathing exercise application, starting a breathing exercise of the breathing exercise application and outputting, during the breathing exercise, breathing instructions to a user of the apparatus;
acquiring a set of heart activity measurement data samples that were measured by a heart activity sensor from the user during the breathing exercise;
computing a set of inter-heartbeat interval samples of the set of heart activity measurement data samples, the inter-heartbeat interval samples characterizing heart rate variability, wherein an inter-heartbeat interval sample represents a time interval between two consecutive heartbeats detected from the heart activity measurement data samples;
computing a dominant frequency of the inter-heartbeat interval samples, and calculating a deviation or variance between said dominant frequency and a reference frequency, and computing a cardiac coherence of the user during the exercise in accordance with said deviation or variance, wherein high deviation or high variance is associated with low cardiac coherence while low deviation or low variance is associated with high cardiac coherence;
the reference frequency being determined as a respiratory frequency of the user at a time of measuring the heart activity measurement samples or determined from user parameters input by the user including at least one or more of the following: age, gender, weight, and body composition;
acquiring a respiratory rate of the user that was measured during the breathing exercise;
computing a score of the breathing exercise on the basis of the respiratory rate and the cardiac coherence, the score indicating a stress level of the user, wherein said computing the score comprises mapping a higher correlation between the measured respiratory rate and the breathing instructions and a higher cardiac coherence to a score value indicating a lower stress level; and
outputting the score through an interface of the apparatus.

2. The apparatus of claim 1, wherein the means are configured to perform said measurement of the heart activity measurement data samples and said output of the breathing instructions simultaneously upon starting the breathing exercise, and to perform, said computing the score of the breathing exercise within the breathing exercise application at least after ending the breathing exercise.

3. The apparatus of any preceding claim, wherein the cardiac coherence represents how harmonic a signal represented by the set of inter-heartbeat interval samples is, higher harmonicity indicating higher cardiac coherence.

4. The apparatus of any preceding claim, wherein the means are configured to compute a momentary score during the exercise on the basis of the respiratory rate and the cardiac coherence, and to output the momentary score through the interface during the exercise.

5. The apparatus of claim 4, wherein the means are configured to define a plurality of zones for the score and to output, through the interface during the breathing exercise, an indicator indicating a zone where the momentary score currently resides as a feedback to the user.

6. The apparatus of any preceding claim, wherein the means are configured to compute the respiratory rate from the set of inter-heartbeat interval samples.

7. The apparatus of any preceding claim, wherein the means are configured to:
estimate the stress level by using a second method where a stress level estimate is computed on the basis of blood pulse wave velocity that was measured from the user and a mapping database (119) defining mapping rules for determining the stress level estimate from the blood pulse wave velocity; and
incorporate the stress level estimate into the score.

8. The apparatus of any preceding claim, wherein the means are configured to:
detect at least one stressor on the basis of measurements conducted on the user; and
execute the breathing exercise as a result of said detecting.

9. The apparatus of any preceding claim, wherein the means comprises:
at least one processor; and
at least one memory including computer program code, the at least one memory and computer program code configured to, with the at least one processor, cause the performance of the apparatus.

10. A computer program product readable by a computer and comprising a computer program code which, when read and executed by the computer, causes the computer to execute a computer process comprising:
executing a breathing exercise application, starting a breathing exercise of the breathing exercise application and outputting, during the breathing exercise, breathing instructions to a user;
acquiring a set of heart activity measurement data samples that were measured by a heart activity sensor from the user during the breathing exercise;
computing a set of inter-heartbeat interval samples of the set of heart activity measurement data samples, the inter-heartbeat interval samples characterizing heart rate variability, wherein an inter-heartbeat interval sample represents a time interval between two consecutive heartbeats detected from the heart activity measurement data samples;
computing a dominant frequency of the inter-heartbeat interval samples, and calculating a deviation or variance between said dominant frequency and a reference frequency, and computing a cardiac coherence of the user during the exercise in accordance with said deviation or variance, wherein high deviation or high variance is associated with low cardiac coherence while low deviation or low variance is associated with high cardiac coherence;
the reference frequency being determined as a respiratory frequency of the user
at a time of measuring the heart activity measurement samples or determined from user parameters input by the user including at least one or more of the following: age, gender, weight, and body composition;
acquiring a respiratory rate of the user that was measured during the breathing exercise;
computing a score of the breathing exercise on the basis of the respiratory rate and the cardiac coherence, the score indicating a stress level of the user, wherein said computing the score comprises mapping a higher correlation between the measured respiratory rate and the breathing instructions and a higher cardiac coherence to a score value indicating a lower stress level; and
outputting the score through an interface.

## Patentansprüche

1. Einrichtung, umfassend Mittel zum Durchführen des Folgenden:
Ausführen einer Atemübungsanwendung, Starten einer Atemübung der Atemübungsanwendung und Ausgeben, während der Atemübung, von Atemanweisungen an einen Benutzer der Einrichtung;
Erlangen eines Satzes von Herzaktivitätsmessdatenproben, die während der Atemübung durch einen Herzaktivitätssensor von dem Benutzer gemessen wurden;
Berechnen eines Satzes von Intervallproben zwischen Herzschlägen aus dem Satz von Herzaktivitätsmessdatenproben, wobei die Intervallproben zwischen Herzschlägen die Herzfrequenzvariabilität charakterisieren, wobei eine Intervallprobe zwischen Herzschlägen ein Zeitintervall zwischen zwei aufeinanderfolgenden, aus den Herzaktivitätsmessdatenproben erfassten Herzschlägen darstellt;
Berechnen einer dominanten Frequenz der Intervallproben zwischen Herzschlägen, und Berechnen einer Abweichung oder Varianz zwischen der dominanten Frequenz und einer Referenzfrequenz, und Berechnen einer Herzkohärenz des Benutzers während der Übung in Übereinstimmung mit der Abweichung oder Varianz, wobei eine hohe Abweichung oder eine hohe Varianz mit einer geringen Herzkohärenz verbunden ist, während eine geringe Abweichung oder eine geringe Varianz mit einer hohen Herzkohärenz verbunden ist;
wobei die Referenzfrequenz als eine Atemfrequenz des Benutzers zum Zeitpunkt des Messens der Herzaktivitätsmessproben bestimmt wird oder aus vom Benutzer eingegebenen Benutzerparametern bestimmt wird, die mindestens eines oder mehrere des Folgenden einschließen: Alter, Geschlecht, Gewicht und Körperbau;
Erlangen einer Atemfrequenz des Benutzers, die während der Atemübung gemessen wurde;
Berechnen einer Punktzahl der Atemübung auf der Grundlage der Atemfrequenz und der Herzkohärenz, wobei die Punktzahl ein Stressniveau des Benutzers angibt, wobei das Berechnen der Punktzahl ein Abbilden einer höheren Korrelation zwischen der gemessenen Atemfrequenz und den Atemanweisungen und einer höheren Herzkohärenz auf einen Punktwert, der ein niedrigeres Stressniveau angibt, umfasst; und
Ausgeben der Punktzahl durch eine Schnittstelle der Einrichtung.

2. Einrichtung nach Anspruch 1, wobei die Mittel dazu konfiguriert sind, die Messung der Herzaktivitätsmessdatenproben und die Ausgabe der Atemübungsanweisungen gleichzeitig beim Starten der Atemübung durchzuführen und die Berechnung der Punktzahl der Atemübung innerhalb der Atemübungsanwendung mindestens nach Beendigung der Atemübung durchzuführen.

3. Einrichtung nach einem vorstehenden Anspruch, wobei die Herzkohärenz darstellt, wie harmonisch ein durch den Satz von Intervallproben zwischen Herzschlägen dargestelltes Signal ist, wobei eine höhere Harmonizität eine höhere Herzkohärenz angibt.

4. Einrichtung nach einem vorstehenden Anspruch, wobei die Mittel dazu konfiguriert sind, während der Übung auf der Grundlage der Atemfrequenz und der Herzkohärenz eine momentane Punktzahl zu berechnen und die momentane Punktzahl während der Übung durch die Schnittstelle auszugeben.

5. Einrichtung nach Anspruch 4, wobei die Mittel dazu konfiguriert sind, eine Vielzahl von Zonen für die Punktzahl zu definieren und während der Atemübung durch die Schnittstelle einen Indikator, der eine Zone angibt, in der sich die momentane Punktzahl derzeit befindet, als eine Rückmeldung an den Benutzer auszugeben.

6. Einrichtung nach einem vorstehenden Anspruch, wobei die Mittel dazu konfiguriert sind, die Atemfrequenz aus dem Satz von Intervallproben zwischen Herzschlägen zu berechnen.

7. Einrichtung nach einem vorstehenden Anspruch, wobei die Mittel zu Folgendem konfiguriert sind:
Schätzen des Stressniveaus durch Verwenden eines zweiten Verfahrens, bei dem eine Stressniveau-Schätzung auf der Grundlage der Blutpulswellengeschwindigkeit, die von dem Benutzer gemessen wurde, und einer Abbildungsdatenbank (119), die Abbildungsregeln zum Bestimmen der Stressniveau-Schätzung aus der Blutpulswellengeschwindigkeit definiert, berechnet wird; und
Einbeziehen der Stressniveau-Schätzung in die Punktzahl.

8. Einrichtung nach einem vorstehenden Anspruch, wobei die Mittel zu Folgendem konfiguriert sind:
Erfassen mindestens eines Stressors auf der Grundlage von an dem Benutzer durchgeführten Messungen; und
Ausführen der Atemübung als ein Ergebnis des Erfassens.

9. Einrichtung nach einem vorstehenden Anspruch, wobei die Mittel umfassen:
mindestens einen Prozessor; und
mindestens einen Speicher, der Computerprogrammcode einschließt, wobei der mindestens eine Speicher und der Computerprogrammcode dazu konfiguriert sind, mit dem mindestens einen Prozessor die Durchführung der Einrichtung zu veranlassen.

10. Computerprogrammprodukt, das durch einen Computer lesbar ist und einen Computerprogrammcode umfasst, der, wenn er durch den Computer gelesen und ausgeführt wird, den Computer veranlasst, einen Computerprozess auszuführen, umfassend:
Ausführen einer Atemübungsanwendung, Starten einer Atemübung der Atemübungsanwendung und Ausgeben, während der Atemübung, von Atemanweisungen an einen Benutzer;
Erlangen eines Satzes von Herzaktivitätsmessdatenproben, die während der Atemübung durch einen Herzaktivitätssensor von dem Benutzer gemessen wurden;
Berechnen eines Satzes von Intervallproben zwischen Herzschlägen aus dem Satz von Herzaktivitätsmessdatenproben, wobei die Intervallproben zwischen Herzschlägen die Herzfrequenzvariabilität charakterisieren, wobei eine Intervallprobe zwischen Herzschlägen ein Zeitintervall zwischen zwei aufeinanderfolgenden, aus den Herzaktivitätsmessdatenproben erfassten Herzschlägen darstellt;
Berechnen einer dominanten Frequenz der Intervallproben zwischen Herzschlägen, und Berechnen einer Abweichung oder Varianz zwischen der dominanten Frequenz und einer Referenzfrequenz, und Berechnen einer Herzkohärenz des Benutzers während der Übung in Übereinstimmung mit der Abweichung oder Varianz, wobei eine hohe Abweichung oder eine hohe Varianz mit einer geringen Herzkohärenz verbunden ist, während eine geringe Abweichung oder eine geringe Varianz mit einer hohen Herzkohärenz verbunden ist;
wobei die Referenzfrequenz als eine Atemfrequenz des Benutzers zum Zeitpunkt des Messens der Herzaktivitätsmessproben bestimmt wird oder aus vom Benutzer eingegebenen Benutzerparametern bestimmt wird, die mindestens eines oder mehrere des Folgenden einschließen: Alter, Geschlecht, Gewicht und Körperbau;
Erlangen einer Atemfrequenz des Benutzers, die während der Atemübung gemessen wurde;
Berechnen einer Punktzahl der Atemübung auf der Grundlage der Atemfrequenz und der Herzkohärenz, wobei die Punktzahl ein Stressniveau des Benutzers angibt, wobei das Berechnen der Punktzahl ein Abbilden einer höheren Korrelation zwischen der gemessenen Atemfrequenz und den Atemanweisungen und einer höheren Herzkohärenz auf einen Punktwert, der ein niedrigeres Stressniveau angibt, umfasst; und
Ausgeben der Punktzahl durch eine Schnittstelle.

## Revendications

1. Appareil comprenant des moyens permettant de réaliser ce qui suit :
exécution d'une application d'exercice respiratoire, démarrage d'un exercice respiratoire de l'application d'exercice respiratoire et production, pendant l'exercice respiratoire, d'instructions respiratoires vers un utilisateur de l'appareil ;
acquisition d'un ensemble d'échantillons de données de mesure d'activité cardiaque qui ont été mesurés par un capteur d'activité cardiaque à partir de l'utilisateur pendant l'exercice respiratoire ;
calcul d'un ensemble d'échantillons d'intervalle entre battements cardiaques de l'ensemble d'échantillons de données de mesure d'activité cardiaque, les échantillons d'intervalle entre battements cardiaques caractérisant la variabilité de fréquence cardiaque, dans lequel un échantillon d'intervalle entre battements cardiaques représente un intervalle de temps entre deux battements cardiaques consécutifs détectés à partir d'échantillons de données de mesure d'activité cardiaque ;
calcul d'une fréquence dominante des échantillons d'intervalle entre battements cardiaques, et calcul d'un écart ou d'une variance entre ladite fréquence dominante et une fréquence de référence, et calcul d'une cohérence cardiaque de l'utilisateur pendant l'exercice en fonction dudit écart ou de ladite variance, dans lequel un écart élevé ou une variance élevée est associé(e) à une faible cohérence cardiaque, tandis qu'un écart faible ou une variance faible est associé(e) à une cohérence cardiaque élevée ;
la fréquence de référence étant déterminée en tant qu'une fréquence respiratoire de l'utilisateur à un moment de mesure d'échantillons de mesure d'activité cardiaque ou déterminée à partir de paramètres d'utilisateur entrés par l'utilisateur incluant au moins un ou plusieurs des éléments suivants : âge, sexe, poids, et composition corporelle ; acquisition d'une fréquence respiratoire de l'utilisateur qui a été mesurée pendant l'exercice respiratoire ;
calcul d'un score de l'exercice respiratoire sur la base de la fréquence respiratoire et de la cohérence cardiaque, le score indiquant un niveau de stress de l'utilisateur, dans lequel ledit calcul du score comprend la mise en correspondance d'une corrélation plus élevée entre la fréquence respiratoire mesurée et les instructions respiratoires et d'une cohérence cardiaque plus élevée avec une valeur de score indiquant un niveau de stress plus faible ; et
production du score à travers une interface de l'appareil.

2. Appareil selon la revendication 1, dans lequel les moyens sont configurés pour réaliser ladite mesure des échantillons de données de mesure d'activité cardiaque et ladite production des instructions respiratoires simultanément lors du démarrage de l'exercice respiratoire, et pour réaliser ledit calcul du score de l'exercice respiratoire dans l'application d'exercice respiratoire au moins après avoir fini l'exercice respiratoire.

3. Appareil selon une quelconque revendication précédente, dans lequel la cohérence cardiaque représente le degré d'harmonicité d'un signal représenté par l'ensemble d'échantillons d'intervalle entre battements cardiaques, une harmonicité plus élevée indiquant une cohérence cardiaque plus élevée.

4. Appareil selon une quelconque revendication précédente, dans lequel les moyens sont configurés pour calculer un score momentané pendant l'exercice sur la base de la fréquence respiratoire et de la cohérence cardiaque, et pour produire le score momentané à travers l'interface pendant l'exercice.

5. Appareil selon la revendication 4, dans lequel les moyens sont configurés pour définir une pluralité de zones pour le score et pour produire, à travers l'interface pendant l'exercice respiratoire, un indicateur indiquant une zone où se trouve actuellement le score momentané en tant qu'une rétroaction à l'utilisateur.

6. Appareil selon une quelconque revendication précédente, dans lequel les moyens sont configurés pour calculer la fréquence respiratoire à partir de l'ensemble d'échantillons d'intervalle entre battements cardiaques.

7. Appareil selon une quelconque revendication précédente, dans lequel les moyens sont configurés pour :
estimer le niveau de stress en utilisant un second procédé où une estimation du niveau de stress est calculée sur la base d'une vitesse d'onde de pulsation sanguine qui a été mesurée à partir de l'utilisateur et d'une base de données de mise en correspondance (119) définissant des règles de mise en correspondance permettant de déterminer l'estimation du niveau de stress à partir de la vitesse d'onde de pulsation sanguine ; et
incorporer l'estimation du niveau de stress dans le score.

8. Appareil selon une quelconque revendication précédente, dans lequel les moyens sont configurés pour :
détecter au moins un facteur de stress sur la base de mesures conduites sur l'utilisateur ;
et
exécuter l'exercice respiratoire à la suite de ladite détection.

9. Appareil selon une quelconque revendication précédente, dans lequel les moyens comprennent :
au moins un processeur ; et
au moins une mémoire incluant un code de programme d'ordinateur, la au moins une mémoire et le code de programme d'ordinateur étant configurés pour, avec le au moins un processeur, amener l'appareil à fonctionner.

10. Produit de programme d'ordinateur lisible par un ordinateur et comprenant un code de programme d'ordinateur qui, lorsque lu et exécuté par l'ordinateur, amène l'ordinateur à exécuter un processus d'ordinateur comprenant :
l'exécution d'une application d'exercice respiratoire, le démarrage d'un exercice respiratoire de l'application d'exercice respiratoire et la production, pendant l'exercice respiratoire, d'instructions respiratoires vers un utilisateur ;
l'acquisition d'un ensemble d'échantillons de données de mesure d'activité cardiaque qui ont été mesurés par un capteur d'activité cardiaque à partir de l'utilisateur pendant l'exercice respiratoire ;
le calcul d'un ensemble d'échantillons d'intervalle entre battements cardiaques de l'ensemble d'échantillons de données de mesure d'activité cardiaque, les échantillons d'intervalle entre battements cardiaques caractérisant la variabilité de fréquence cardiaque, dans lequel un échantillon d'intervalle entre battements cardiaques représente un intervalle de temps entre deux battements cardiaques consécutifs détectés à partir d'échantillons de données de mesure d'activité cardiaque ;
le calcul d'une fréquence dominante des échantillons d'intervalle entre battements cardiaques, et le calcul d'un écart ou d'une variance entre ladite fréquence dominante et une fréquence de référence, et le calcul d'une cohérence cardiaque de l'utilisateur pendant l'exercice en fonction dudit écart ou de ladite variance, dans lequel un écart élevé ou une variance élevée est associé(e) à une faible cohérence cardiaque, tandis qu'un écart faible ou une variance faible est associé(e) à une cohérence cardiaque élevée ;
la fréquence de référence étant déterminée en tant qu'une fréquence respiratoire de l'utilisateur à un moment de mesure d'échantillons de mesure d'activité cardiaque ou déterminée à partir de paramètres d'utilisateur entrés par l'utilisateur incluant au moins un ou plusieurs des éléments suivants : âge, sexe, poids, et composition corporelle ; l'acquisition d'une fréquence respiratoire de l'utilisateur qui a été mesurée pendant l'exercice respiratoire ;
le calcul d'un score de l'exercice respiratoire sur la base de la fréquence respiratoire et de la cohérence cardiaque, le score indiquant un niveau de stress de l'utilisateur, dans lequel ledit calcul du score comprend la mise en correspondance d'une corrélation plus élevée entre la fréquence respiratoire mesurée et les instructions respiratoires et d'une cohérence cardiaque plus élevée avec une valeur de score indiquant un niveau de stress plus faible ; et
la production du score à travers une interface.
